# FASCICULE DE BREVET EUROPEEN

(11) **EP 0 443 925 B1**
(45) Date de publication et mention de la délivrance du brevet: **23.08.1995**
(21) Numéro de dépôt: 91400413.0
(22) Date de dépôt: 18.02.1991
(51) Int. Cl.: C12P 7/26

(54) **Procédé d'obtention d'irone par voie microbiologique**
Verfahren zur mikrobiologischen Herstellung von Ironen
Process for the microbiological obtention of irones

(30) Priorité: 22.02.1990 FR 9002213
(43) Date de publication de la demande: 28.08.1991
(73) Titulaire: ELF AQUITAINE, 92400 Courbevoie (FR)
(72) Inventeur: Gil, Gérard, F-13400 Aubagne (FR); Le Petit, Jean, F-13190 Allauch (FR)
(74) Mandataire: Gillard, Marie-Louise

(56) Documents cités:
- CHEMICAL ABSTRACTS, vol. 110, 1989, Columbus, OH (US); R.M. BAGDASAROVA et al., p. 504, no. 73846e#

## Description

La présente invention concerne un procédé de préparation d'irone, dont les mélanges des trois isomères, α, β et γ, sont utilisés dans l'industrie alimentaire et celle des parfums et des cosmétiques, pour leur odeur de violette.

Lors de leur récolte, les rhizomes d'iris ne contiennent pratiquement pas d'irone, et c'est au cours de leur stockage prolongé que celle-ci se forme, vraisemblablement par transformation oxydative de triterpènes initialement présents.

Dans le procédé habituel d'obtention d'irone, il faut donc attendre plus de deux ans après la récolte des rhizomes pour extraire une quantité raisonnable d'irone par entraînement à la vapeur ou dans un solvant organique.

Récemment, il a été proposé dans la demande FR-A-2 620 702 un procédé qui supprime le stockage prolongé, procédé caractérisé en ce que les précurseurs de l'irone sont extraits des rhizomes, peu après leur récolte, par un solvant lipophile puis soumis à une oxydation chimique, qui libère les molécules d'irone.

Ce procédé présente l'inconvénient de mettre en oeuvre des réactifs chimiques qui interdisent l'emploi de l'irone ainsi obtenue dons l'industrie alimentaire et il était souhaitable de trouver un procédé naturel de dégradation, tel qu'un procédé micro-biologique. Néanmoins, il n'était pas évident de trouver des micro-organismes susceptibles d'effectuer cette transformation, comme le font les enzymes végétales au cours du stockage.

On a maintenant trouvé des micro-organismes aérobies qui dégradent les précurseurs, sans dégrader simultanément de façon appréciable l'irone formée, en quelques jours et avec des rendements en irone par rapport à la quantité de rhizomes d'iris mis en oeuvre nettement supérieurs à ceux obtenus avec le procédé classique, ce qui est d'autant plus intéressant que la quantité de rhizomes d'iris disponible sur le marché européen diminue actuellement, sans diminution de la demande en irone.

Le procédé selon l'invention consiste à transformer les précurseurs de l'irone présents dans les rhizomes d'iris frais en les soumettant à l'action d'un micro-organisme du genre Botryotinia encore appelé Sclerotinia, dont la forme imparfaite est dénommée Botrytis. Ces champignons sont décrits dans l'ouvrage de M.B. Ellis - Hyphomycetes p. 178-184 (1971) - Commonwealth Mycological Institute.

Une espèce courante de ce champignon filamenteux, dont on peut se procurer diverses souches dans les collections internationales de micro-organismes, est la Botryotinia convulata, mais d'autres espèces, telles que B. draytonii, B. ficariarum ou B. fuckeliana peuvent convenir; celles-ci sont décrites dans : Microfungi on land plants. An Identification Handbook - M.B. Ellis et J.P. Ellis - 1985 - Croom Helm - Londres.

Le micro-organisme peut être utilisé entier dans son milieu de culture ou seulement le mycélium; on peut aussi utiliser le milieu dans lequel a été cultivé le champignon, après l'élimination du mycélium.

Le milieu de culture peut être l'un de ceux généralement utilisés pour ce type de champignons, c'est-à-dire de l'eau contenant une source de carbone, une source d'azote, et des sels métalliques. Comme source de carbone, on peut citer les sucres, tels que le glucose, le maltose ou le lactose ou encore le glycérol; comme source d'azote, on peut citer la caséine, des aminoacides ou des peptides et protéines dont la gélatine et la gélatine hydrolysée, un extrait de malt ou de rafle de maïs, un extrait de viande, de l'urée, ou de la farine de soja ; comme sels métalliques, on peut citer les phosphates, les citrates ou les acétates de sodium, magnésium, cuivre, manganèse ou fer et, mieux, un mélange de sels de fer, de cuivre et de manganèse. Le pH du milieu de culture est acide ou neutre, compris entre 3,5 et 7 et, de préférence, entre 5,5 et 6; on peut éventuellement fixer le pH du milieu avec un tampon à base de phosphate, phtalate ou Tris ou ajuster le pH au cours du développement du champignon. La température du milieu lors de la culture est celle la plus favorable au développement du micro-organisme; elle est en général comprise entre 15°C et 30°C, et la réaction de transformation des précurseurs de l'irone sera de préférence effectuée à cette température.

L'étape préalable d'extraction des précurseurs des rhizomes frais, éventuellement séchés, notamment pour faciliter leur transport, est effectuée par mise en suspension des rhizomes concassés ou broyés dans un solvant organique généralement utilisé dans l'industrie des arômes, tel que les solvants alcooliques, méthanol ou éthanol, les solvants chlorés, chlorure de méthylène ou dichloroéthane, les hydrocarbures aliphatiques ou aromatiques, hexane ou benzène; on peut éventuellement utiliser des mélanges de solvants ou faire deux extractions successives avec des solvants différents, par exemple avec un solvant miscible à l'eau et l'autre non. Il est préférable d'utiliser des solvants alcooliques ou chlorés et, mieux, le méthanol et le chlorure de méthylène. On peut éliminer les composés hydrosolubles entraînés lors d'une première extraction, en redissolvant l'extrait dans un solvant non miscible à l'eau et en lavant cette phase organique, avant d'éliminer le solvant pour obtenir l'extrait qui sera soumis à l'action du champignon.

On peut utiliser des Iris germanica, pallida ou florentina de diverses provenances, comme le Maroc ou l'Italie. On sait que le rendement en irone et la proportion des isomères dans le produit obtenu peuvent varier sensiblement avec l'espèce, la provenance et la maturité des iris mis en oeuvre; dans tous les cas, le procédé selon l'invention donne des résultats nettement plus avantageux que le procédé classique.

Selon l'invention, on introduit l'extrait, de préférence en suspension dans l'eau ou en solution dans un petit volume d'un solvant organique hydrophile tel que l'acétone, un alcool ou le dioxanne, dans le milieu où on a cultivé le champignon jusqu'à obtenir un maximum de biomasse, soit environ 48 h, à raison de 1 g à 20 g d'extrait par litre de milieu; étant donné l'insolubilité de l'extrait dans le milieu, comme il est courant dans ce domaine, on peut introduire simultanément une faible quantité d'un agent tensioactif comme un ester de sorbitol et d'acide gras tel que les Tween®, ainsi qu'un agent antimousse comme une huile de silicone, un polyalkylèneglycol, de l'huile de soja ou de maïs. Le milieu est maintenu sous agitation et éventuellement aéré par exemple pendant 20 à 60 h à 20°C; on élimine alors les corps cellulaires par filtration et on sépare l'irone formée par entraînement à la vapeur ou on l'extrait dans un solvant organique non miscible à l'eau. La durée et la température optimales de la réaction dépendent des conditions opératoires : matériel, nature et concentration des réactifs, espèce et souche de champignons et le spécialiste sera à même de déterminer par des essais préalables les paramètres optimaux de la réaction, sachant que l'irone formée peut être dégradée par le champignon; le déroulement de la réaction peut, notamment, être suivi en chromatographie en phase gazeuse.

La biomasse nécessaire pour transformer une quantité donnée d'extrait sera inférieure si, selon une technique d'induction connue, on introduit l'extrait dans le milieu de préculture et de culture du champignon, en faible concentration, de 0,05% à 0,1%.

Par ailleurs, les rendements en irone sont nettement améliorés, si on ajoute en fin de culture, pour perméabiliser les membranes cellulaires, un surfactant non ionique, comme les esters de polyols et d'acide gras, tel que le Tween®, ou les polyalkylène-glycols, tel que l'octylphénoxypolyéthoxyéthanol ou Triton® X, avant d'introduire l'extrait à dégrader. Ces agents sont utilisés, en général, à une concentration de 0,05% à 0,25%.

Selon un autre aspect de l'invention, on peut séparer la biomasse du milieu de culture par filtration avant d'y introduire l'extrait, éventuellement après avoir perméabilisé les membranes.

On peut aussi, mais les rendements de transformation sont moins bons, séparer le mycélium de son milieu de culture, éventuellement après perméabilisation des membranes, et le remettre en suspension dans une solution aqueuse tamponnée, de préférence contenant 5 à 15 g/l de glucose, et introduire dans ce milieu, de préférence en présence d'un agent tensioactif, l'extrait à transformer.

Selon un autre aspect de l'invention, le procédé est mis en oeuvre avec des micro-organismes immobilisés, ce qui peut permettre d'opérer en continu; l'immobilisation est effectuée, de façon classique, soit en cultivant le champignon dans un milieu contenant un solide, sous forme de billes, de pastilles, d'anneaux ou autre forme en verre ou en polymère inerte, sur lequel le mycélium en croissance se fixera, soit en séparant le champignon ou le mycélium de son milieu de culture et en l'incorporant dans des matrices poreuses, formées par réticulation chimique de monomères convenables ou par coacervation de polymères.

Dans ce qui suit, on décrit des exemples de mise en oeuvre de l'invention.

On illustre tout d'abord l'extraction des précurseurs :

2,5 kg de rhizomes d'iris Pallida d'Italie, séchés mais contenant encore 60 % d'eau, sont nettoyés et mis dans un récipient en inox de 60 l comprenant un bloc rotatif central à lames avec 24 l d'éthanol à 96 % ; le mélange est maintenu 20 h à la température de reflux du solvant, puis ramené à température ambiante et filtré ; le filtrat est concentré sous vide à siccité ; les solides séparés sont remis dans le récipient avec 12 l de chlorure de méthylène et le mélange est maintenu pendant 20 h à la température de reflux du solvant, puis filtré à température ambiante, le filtrat est concentré sous vide à siccité et le résidu, réuni au précédent, est redissous dans 2 l de chlorure de méthylène. La phase organique est lavée deux fois à l'eau et évaporée à siccité pour donner 1 kg d'extrait sec.

On illustre ensuite l'action du micro-organisme. Des souches de Botryotinia convulata disponibles dans la collection du Centraalbureau Voor Schlimmelcultures Baarn (CBS), Delft (Pays-Bas), sous les références 285-38, 286-38, 428-38, ont été utilisées.

### EXEMPLE 1

### Culture du micro-organisme

Le champignon conservé dans un milieu PDA (Potato Dextrose Agar), commercialisé par Merieux (France), est utilisé pour ensemencer 100 ml d'un milieu de préculture de pH 5,7, constitué de 15 g d'extrait de malt par litre d'eau, 10 g/l de glucose, 2 mg/l de sulfate de cuivre, 2 mg/l de citrate de fer et d'ammonium et 2 mg/l de chlorure de zinc avec une goutte d'une solution acétonique de l'extrait; après 48 h sous agitation, à 20°C, la préculture est introduite dans 800 ml du milieu de culture de pH 5,7 constitué de 25 g/l d'eau de liqueur de maïs (corn steep liquor) et de 2 mg/l de sulfate de cuivre, de citrate de fer et d'ammonium et de chlorure de zinc, avec 1 ml d'une solution acétonique d'extrait. Après 48 h d'agitation à 20°C, on introduit dans le milieu du Triton® X 100 à la concentration de 0,1% puis, 2 h après, 5 g d'extrait en émulsion dans 10 ml d'eau et 1 ml d'acétone contenant 0,2% de Tween® 80. Le milieu est incubé à 20°C, puis l'irone est séparée par entraînement à la vapeur. La quantité d'irone obtenue après des essais ayant duré de 12 h à 72 h, mesurée par chromatographie en phase gazeuse, figure dans le tableau 1; elle est exprimée en gramme d'irone par kilo de matière sèche de rhizomes mis en oeuvre pour préparer l'extrait traité.

**TABLEAU 1**

| Durée | g d'irone/kg d'extrait sec de rhizome |
|---|---|
| 12 h | 1,15 |
| 24 h | 1,2 |
| 36 h | 2,2 |
| 48 h | 2,3 |
| 72 h | 1,1 |

### EXEMPLE 2

On répète le même mode opératoire qu'à l'exemple 1, mais sans introduire de Triton® en fin de culture. On obtient alors seulement 1,1 g d'irone au bout de 48 h.

## Revendications

1. Procédé d'obtention d'irone, caractérisé en ce qu'on soumet les précurseurs extraits des rhizomes d'iris frais à l'action d'un champignon du genre Botryotinia.

2. Procédé selon la revendication 1, caractérisé en ce que le champignon est de l'espèce Botryotinia convulata.

3. Procédé selon l'une des revendications 1 ou 2, caractérisé en ce qu'un sel de fer est introduit dans le milieu de culture du champignon.

4. Procédé selon l'une quelconque des revendications précédentes, caractérisé en ce que le pH du milieu de culture est compris entre 5,5 et 6.

5. Procédé selon l'une quelconque des revendications précédentes, caractérisé en ce que l'on effectue une induction du champignon par addition de traces de l'extrait de rhizome dans le milieu de préculture et de culture du champignon.

6. Procédé selon l'une quelconque des revendications précédentes, caractérisé en ce qu'on traite le champignon par un agent perméabilisant des membranes avant de le mettre en contact avec les précurseurs.

7. Procédé selon la revendication 6, caractérisé en ce que l'agent perméabilisant est le Triton® X.

8. Procédé selon l'une quelconque des revendications précédentes, caractérisé en ce que les précurseurs sont extraits des rhizomes par un solvant choisi parmi les solvants alcooliques, les solvants chlorés, les hydrocarbures aliphatiques ou aromatiques et leurs mélanges.

9. Procédé selon l'une quelconque des revendications précédentes, caractérisé en ce que les précurseurs sont extraits des rhizomes par un solvant choisi parmi le méthanol, l'éthanol et le chlorure de méthylène.

## Claims

1. Process for obtaining irone, characterized in that the precursors extracted from fresh iris rhizomes are subjected to the action of a fungus of the genus Botryotinia.

2. Process according to claim 1, characterized in that the fungus belongs to the species Botryotinia convulata.

3. Process according to one of claims 1 or 2, characterized in that an iron salt is introduced into the culture medium of the fungus.

4. Process according to any one of the preceding claims, characterized in that the pH of the culture medium lies between 5.5 and 6.

5. Process according to any one of the preceding claims, characterized in that the fungus growth is induced by addition of small amounts of the rhizome extract to the preculture and culture medium of the fungus.

6. Process according to any one of the preceding claims, characterized in that the fungus is treated with an agent for making the membranes permeable before it is placed in contact with the precursors.

7. Process according to claim 6, characterized in that the permeabilizing agent is Triton® X.

8. Process according to any one of the preceding claims, characterized in that the precursors are extracted from the rhizomes by a solvent selected from alcoholic solvents, chlorinated solvents, aromatic or aliphatic hydrocarbons and their mixtures.

9. Process according to any one of the preceding claims, characterized in that the precursors are extracted from the rhizomes by a solvent selected from methanol, ethanol and methylene chloride.

## Patentansprüche

1. Verfahren zur Herstellung von Ironen, dadurch gekennzeichnet, daß die aus frischen Iris-Rhizomen extrahierten Vorläuferverbindungen der Einwirkung eines Pilzes der Gattung Botryotinia unterworfen werden.

2. Verfahren nach Anspruch 1, dadurch gekennzeichnet, daß ein Pilz der Art Botryotinia convulata verwendet wird.

3. Verfahren nach Anspruch 1 oder 2, dadurch gekennzeichnet, daß ein Eisensalz in das Kulturmedium des Pilzes eingebracht wird.

4. Verfahren nach einem der vorhergehenden Ansprüche, gekennzeichnet durch einen pH-Wert des Kulturmediums von 5,5 bis 6.

5. Verfahren nach einem der vorhergehenden Ansprüche, dadurch gekennzeichnet, daß eine Induktion des Pilzes durch Zusatz von Spuren von Rhizomextrakt zum Vorkulturmedium und zum Kulturmedium des Pilzes vorgenommen wird.

6. Verfahren nach einem der vorhergehenden Ansprüche, dadurch gekennzeichnet, daß der Pilz vor dem Inkontaktbringen mit den Vorläuferverbindungen mit einem Mittel zur Membranpermeabilisierung behandelt wird.

7. Verfahren nach Anspruch 6, dadurch gekennzeichnet, daß als Mittel zur Membranpermeabilisierung Triton® X verwendet wird.

8. Verfahren nach einem der vorhergehenden Ansprüche, dadurch gekennzeichnet, daß die Vorläuferverbindungen mit einem unter alkoholischen Lösungsmitteln, chlorierten Lösungsmitteln, aliphatischen oder aromatischen Kohlenwasserstoffen sowie deren Gemischen ausgewählten Lösungsmittel aus den Rhizomen extrahiert werden.

9. Verfahren nach einem der vorhergehenden Ansprüche, dadurch gekennzeichnet, daß die Vorläuferverbindungen mit einem unter Methanol, Ethanol und Methylenchlorid ausgewählten Lösungsmittel aus den Rhizomen extrahiert werden.
